# EUROPÄISCHE PATENTSCHRIFT

(11) **EP 4 255 720 B1**
(45) Veröffentlichungstag und Bekanntmachung des Hinweises auf die Patenterteilung: **29.01.2025**
(21) Anmeldenummer: 21836062.6
(22) Anmeldetag: 07.12.2021
(51) Int. Cl.: B29C 65/08, A61F 13/15, B29L 31/48

(54) **BEARBEITUNGSELEMENT MIT STRUKTURELEMENT**
MACHINING ELEMENT WITH STRUCTURAL ELEMENT
ÉLÉMENT D'USINAGE AVEC ÉLÉMENT DE STRUCTURE

(30) Priorität: 07.12.2020 DE 102020132522
(43) Veröffentlichungstag der Anmeldung: 11.10.2023
(73) Patentinhaber: Herrmann Ultraschalltechnik GmbH & Co. KG, 76307 Karlsbad (DE)
(72) Erfinder: ROUSSEL-GARCIA, Raquel, 75180 Pforzheim (DE); BODE, Robin Alexander, 75180 Pforzheim (DE); ZINK, Timo, 76139 Karlsruhe (DE)
(74) Vertreter: WSL Patentanwälte Partnerschaft mbB
(86) Internationale Anmeldenummer: PCT/EP2021/084609
(87) Internationale Veröffentlichungsnummer: WO 2022/122736

(56) Entgegenhaltungen:
- EP-A1- 0 736 356
- EP-B1- 0 531 666
- EP-B1- 1 477 293
- EP-B1- 2 063 842
- EP-B1- 3 094 468
- EP-B1- 3 209 433
- WO-A1-2018/064340
- WO-A1-2022/097455
- DE-A1- 2 154 057
- US-A1- 2010 243 172

## Beschreibung

Die vorliegende Erfindung betrifft ein Bearbeitungselement für die Ultraschallbearbeitung ei-nes Materials, wie z.B. eine Sonotrode oder ein Amboss. Ein entsprechendes Bearbeitungselement wird beispielsweise in EP 3 209 433 B1 und WO 2018/064340 A1 beschrieben.

Gerade bei der Verbindung von Vliesstoffmaterialien kommt zunehmend Ultraschall zur Anwendung. Dabei werden zwei miteinander zu verbindende Vliesstoffabschnitte übereinander in einen Spalt zwischen einer Sonotrode und einem Amboss gebracht und die Sonotrode mit einer Ultraschallschwingung beaufschlagt. An den aufeinander liegenden Kontaktflächen kommt es aufgrund der durch die Ultraschallschwingung induzierten Reibung zu einer punktuellen Erwärmung, sodass insbesondere thermoplastische Bestandteile des Vliesstoffes aufgeschmolzen werden. Die aufgeschmolzenen Bestandteile von zu verbindenden Materialabschnitten fließen ineinander und sorgen nach deren Abkühlung für eine feste Verbindung. So ist es möglich, bei der Herstellung von Windeln entsprechende Vliesstoffabschnitte zur Ausbildung einer Seitennaht miteinander zu verbinden.

Häufig ist bei der Bearbeitung von Vliesstoffen auch eine Raffung des Materials erwünscht. Hierzu werden zusätzliche elastische Fäden zwischen die zu verbindenden Vliesstoffabschnitte eingebracht. Die zu verbindenden Vliesstoffabschnitte werden dann an mindestens zwei Verbindungsflächen miteinander verbunden, wobei ein Faden während einem Bearbeitung mittels Ultraschall zwischen den beiden Verbindungsflächen derart fixiert wird, dass eine formschlüssige Verbindung zwischen Faden und Vliesstoffabschnitten in zwei Raumrichtungen, die senkrecht zueinander orientiert sind, vorliegt. Auf diese Weise kann eine Raffung des Material erzielt werden.

Dabei kann das Bearbeitungselement eine im Wesentlichen zylinderförmige oder zylindersegmentförmige Trägerfläche aufweisen, die dafür vorgesehen ist, während der Bearbeitung mit dem Material in Kontakt zu treten. Das Bearbeitungselement wird dann während der Bearbeitung um seine Längsachse gedreht, sodass die Trägerfläche auf dem zu bearbeitenden Material abrollt.

Dabei weist die Trägerfläche oft zumindest ein Strukturelement auf, welches in radialer Richtung über der Trägerfläche vorsteht, so dass das Strukturelement eine Oberseite aufweist, die dafür vorgesehen ist, mit dem zu bearbeitenden Material in Kontakt zu treten. Die eigentliche Verschweißung erfolgt dann in dem Bereich zwischen der Oberseite des Strukturelementes und einer Siegelfläche eines hierzu beabstandet angeordneten Gegenelementes.

Im Falle der Fadenfixierung erstreckt sich das längliche Strukturelement typischerweise entlang der Längsachse des Bearbeitungselements, sodass das Strukturelement üblicherweise in einem Winkel, zumeist einem rechten Winkel, zu der Orientierung des Fadens ausgerichtet ist. Durch das Strukturelement wird der Faden daher abschnittsweise mit den Vliesstoffabschnitten verbunden. Bereiche, in denen der Faden frei beweglich ist, wechseln sich ab mit Bereichen, in denen der Faden mit den Vliesstoffabschnitten verbunden ist. Diese Verbindung kann entweder durch eine kraftschlüssige Verbindung oder durch eine stoffschlüssige Verbindung zwischen dem Vliesstoff und dem Faden in der Raumrichtung, in welcher keine formschlüssige Verbindung vorliegt, erzielt werden. Ist der Faden während der Ultraschallbearbeitung gedehnt, so entsteht durch die abschnittsweise Fixierung des Fadens eine Raffung des Vliesstoffes, wenn der Faden nach der Bearbeitung entspannt wird.

Beispielsweise kann das Gegenelement eine Sonotrode und das Bearbeitungselement ein Amboss sein. Im Folgenden wird die Erfindung anhand dieses Beispiels erläutert, da dies die bevorzugte Ausführungsform ist. Prinzipiell ist es aber möglich, das Bearbeitungselement als Sonotrode und das Gegenelement als Amboss auszubilden.

Während der Bearbeitung rollt die Trägerfläche mit den Strukturelementen auf dem zu bearbeitenden Material ab, so dass insbesondere die Strukturelemente eine Verschweißung bewirken.

Die Verarbeitungsgeschwindigkeit ist mit den Vorrichtungen des Standes der Technik begrenzt.

Zwar lässt sich grundsätzlich die Vorschubgeschwindigkeit, d.h. die Geschwindigkeit, mit welcher das Material durch den Spalt zwischen Bearbeitungselement und Gegenelement bewegt wird, erhöhen.

Allerdings wird dann durch die Sonotrode, die mit einer festen Frequenz auf das Material einwirkt, nicht mehr genügend Energie in das Material eingebracht, um eine zuverlässige Verschweißung zu ermöglichen. Dies liegt daran, dass bei einer höheren Vorschubgeschwindigkeit das Material kürzer mit der Siegelfläche der Sonotrode in Kontakt tritt und daher weniger "Schläge" von der Sonotrode auf das Material aufgebracht werden.

Dies kann teilweise dadurch kompensiert werden, dass die Kraft, mit welcher die Sonotrode auf das zu bearbeitende Material gedrückt wird, erhöht wird. Dadurch wird "pro Schlag" der Sonotrode mehr Energie in das Material übertragen. Dies sorgt allerdings für eine höhere Reibung und führt dazu, dass die aufgeschmolzenen Bestandteile, die sich durch die Ultraschallbearbeitung an den Grenzflächen zwischen den zu verschweißenden Materialschichten ausbilden. d. h. in der sogenannten Fügezone, durch die Strukturelemente aus der Fügezone gedrückt werden, was ebenfalls zu einer schlechteren Naht führt, da nicht mehr genug thermoplastische Bestandteile in der Fügezone zur Verfügung stehen. Alternativ oder in Kombination könnte auch die Schwingungsamplitude der Ultraschallschwingung erhöht werden. Auch hierdurch wird "pro Schlag" der Sonotrode mehr Energie in das Material übertragen. Diese ist jedoch nur begrenzt möglich. Wird die Sonotrode mit zu hoher Schwingungsamplitude betrieben, kann es zu Beschädigungen des Sonotrodenmaterials kommen.

Um eine höhere Verarbeitungsgeschwindigkeit zu erzielen, sind bereits sogenannte "welding wheels" verwendet worden, bei welchen mehrere Sonotroden auf einem Rad angeordnet sind, um die Kontaktzeit während der Drehbewegung des Rades zu erhöhen. Diese Lösung ist jedoch sehr aufwendig.

Sollen außerdem Fäden mit den Vliesstoffen verbunden werden, besteht zusätzlich die Problematik, dass zu schmale Strukturelemente eine nicht ausreichende Fadenklemmung zur Folge haben, da die Klemmkraft aufgrund der geringen Wechselwirkungsfläche, die der Oberseite der Strukturelemente entspricht, mit den Vliesstoffabschnitten reduziert ist. Wird das Strukturelement hingegen zu breit gewählt, so ist die Wechselwirkungsfläche und damit die Klemmkraft größer, allerdings führt die größere Wechselwirkungsfläche auch zu einer erhöhten Reibung mit dem zu bearbeitenden Material. Weiterhin wird bei zu breiten Strukturelementen der Freiraum, in welchem sich der Faden ungehindert bewegen kann, reduziert, was sich negativ auf die Raffeigenschaften des Materials auswirkt.

Ausgehend von dem beschriebenen Stand der Technik ist es daher Aufgabe der vorliegenden Erfindung, ein Bearbeitungselement anzugeben, mit welchem eine zuverlässige Verschwei-βung ermöglicht wird.

Erfindungsgemäß wird diese Aufgabe dadurch gelöst, dass die Oberseite einen Basisabschnitt und zumindest einen Ausnehmungsabschnitt aufweist, welcher einen geringeren Abstand von der Längsachse hat als der Basisabschnitt, wobei in einer Schnittansicht senkrecht zur Längsachse Basisabschnitt und Ausnehmungsabschnitt nebeneinander angeordnet sind, wobei die durch den Ausnehmungsabschnitt gebildete Ausnehmung sich nicht bis zur Trägerfläche erstreckt, wobei vorzugsweise die Ausnehmung eine Tiefe von weniger als 1 mm und besonders bevorzugt zwischen 0,05 mm und 0,2 mm hat, in Übereinstimmung mit Anspruch 1.

Bei dem Abrollen des Strukturelementes auf dem Material können plastifizierte Bestandteile im Ausnehmungsabschnitt aufgenommen werden, so dass das beschriebene Herausdrücken der plastifizierten Bestandteile aus der Fügezone verringert wird.

Im Falle der Fadenfixierung wird durch die Konzentration der Schmelze in dem Ausnehmungsabschnitt die Fadenklemmung zusätzlich verbessert.

Der Ausnehmungsabschnitt führt weiterhin dazu, dass die effektive Wechselwirkungsfläche und damit die Reibung mit dem Material reduziert wird. Gleichzeitig kann das Strukturelement breiter ausgestaltet werden, sodass der Faden ausreichend fixiert wird. Weiterhin führt der Ausnehmungsabschnitt dazu, dass weniger Kraft zwischen Bearbeitungselement und Gegenelement aufgebracht werden muss, um das gleiche Bearbeitungsergebnis zu erzielen. Zudem wurde eine verbesserte Haptik des resultierenden Produktes festgestellt.

In einer weiteren bevorzugten Ausführungsform ist der Ausnehmungsabschnitt als Rille, welche möglichst nicht allein in Umfangsrichtung ausgerichtet ist, ausgebildet. Falls die Rille in Umfangsrichtung ausgerichtet ist, umläuft die Rille vorzugsweise die Trägerfläche nicht vollumfänglich, sondern erstreckt sich nur über einen Umfangswinkel < 360° und zwar am besten über einen Umfangswinkel von weniger als 45° und am besten von weniger als 25°. Es ist auch möglich, dass mehrere Rillen in Umfangsrichtung mit Abstand voneinander angeordnet sind.

Die Rille soll die Verschweißung nicht unterbrechen, sondern lediglich aufgeschmolzenes Material aufnehmen, sodass es im Wesentlichen an Ort und Stelle verbleibt und zur Verbindung der Materialschichten dienen kann.

Es hat sich gezeigt, dass diese Rille das nachteilige Delokalisieren der Schmelze aufgrund des erhöhten Druckes der Sonotrode auf das zu bearbeitende Material, an der Stelle der Rille stoppen kann. Die Schmelze wird dann nur bis zu der Rille bewegt. Die Rille dient somit als Aufnahme für das Schmelzematerial.

In einer bevorzugten Ausführungsform hat die Rille eine Breite, die kleiner als 1 mm ist und vorzugsweise kleiner als 0,6 mm ist. Am besten beträgt die Breite der Rille zwischen 0,2 und 0,4 mm.

In Abhängigkeit von dem zu verschweißenden Material kann es ausreichend sein, wenn die Rille eine Querschnittsfläche von weniger als 0,15 mm² hat. Vorzugsweise ist die Querschnittsfläche sogar geringer als 0,05 mm² und am besten liegt die Querschnittsfläche zwischen 0,015 mm² und 0,04 mm².

In einer bevorzugten Ausführungsform weist das Strukturelement eine Mehrzahl von Rillen, vorzugsweise mindestens drei Rillen, in der Oberseite auf, welche nicht in Umfangsrichtung ausgerichtet sind, wobei vorzugsweise die Rillen parallel zueinander angeordnet sind. Durch die Rillen gelingt es, aufgeschmolzenes Material an der jeweiligen Position zu halten, daher sind mehrere Rillen von Vorteil.

In einer weiteren bevorzugten Ausführungsform ist vorgesehen, dass die Oberseite des Strukturelementes einen Hauptabschnitt, der im Wesentlichen eben oder mit einer konvexen Krümmung mit einem Krümmungsradius, der dem Abstand des Hauptabschnittes von der Zylinderachse entspricht, ausgebildet ist, und mindestens einen sich an den Hauptabschnitt in Umfangsrichtung anschließenden Fasenabschnitt aufweist. Der Fasenabschnitt ist entweder gegenüber dem Hauptabschnitt abgewinkelt, sodass Haupt- und Fasenabschnitt einen Winkel kleiner als 180° einschließen, oder der Fasenabschnitt ist konvex gekrümmt, wobei, wenn der Hauptabschnitt konvex gekrümmt ausgebildet ist, der Krümmungsradius des Fasenabschnittes kleiner als der Krümmungsradius des Hauptabschnittes ist. Dabei ist vorzugsweise der mindestens eine Ausnehmungsabschnitt im Hauptabschnitt angeordnet ist. Der Fasenabschnitt dient dazu, das Material allmählich auf den schweißenden Kontakt zwischen dem Hauptabschnitt und dem Gegenelement vorzubereiten. Am Übergang zwischen Haupt- und Fasenabschnitt ändert sich die Steigung oder die Krümmung der Oberseite. Dadurch ist sichergestellt, dass bei der Verwendung des Bearbeitungselementes der Abstand zwischen Strukturelement und Gegenelement kontinuierlich kleiner wird, bis der kleinste Abstand zwischen Strukturelement und Gegenelement verwirklicht worden ist.

In einer weiteren bevorzugten Ausführungsform ist vorgesehen, dass die Oberseite zwei sich in Umfangsrichtung an gegenüberliegenden Seiten an den Hauptabschnitt anschließende Fasenabschnitte aufweist, die gegenüber dem Hauptabschnitt abgewinkelt sind, sodass Haupt- und Fasenabschnitt jeweils einen Winkel kleiner 180° einschließen. Bei der Bearbeitung hat somit die Oberseite des Strukturelementes nicht nur einen einlaufenden Fasenabschnitt, sondern zudem einen auslaufenden Fasenabschnitt, wodurch auch am Ende der Bearbeitung des Strukturelementes die vom Gegenelement auf das Bearbeitungselement aufgebrachte Kraft nur allmählich reduziert wird.

In einer weiteren bevorzugten Ausführungsform hat die Oberseite des Strukturelementes eine längliche Form mit einer Länge l und einer Breite b, wobei l > b ist. Dabei erstreckt sich die Länge vorzugsweise nicht parallel zur Längsachse, sondern am besten im Wesentlichen senkrecht hierzu.

In einer weiteren Ausführungsform erstreckt sich das Strukturelement sowie der in der Oberseite angeordnete Ausnahmeabschnitt kontinuierlich über eine gesamte Länge l des Bearbeitungselements, wobei die Länge l im Wesentlichen parallel zu der Längsachse orientiert ist. Dabei gilt vorzugsweise, dass die Länge l des Strukturelementes deutlich größer ist als die Breite b des Strukturelements, die im Wesentlichen senkrecht zu der Länge l angeordnet ist. In einer weiteren Ausführungsform erstreckt sich das Strukturelement ebenfalls im Wesentlichen entlang der Längsachse des Bearbeitungselements, wobei das Strukturelement sowie der in der Oberseite angeordnete Ausnahmeabschnitt schlangenlinienförmig verlaufen. Es versteht sich, dass in diesem Fall der Basisabschnitt ebenfalls schlangenlinienförmig verläuft.

Die vorliegende Erfindung betrifft auch eine Ultraschallschweißvorrichtung gemäß Anspruch 11, mit einem Bearbeitungselement, wie es gerade beschrieben wurde. Diese Ultraschallschweißvorrichtung weist zusätzlich zu dem Bearbeitungselement ein Gegenelement auf, das eine Siegelfläche aufweist, die gegenüberliegend zu dem Bearbeitungselement angeordnet werden kann, sodass sich zwischen der Oberseite des Strukturelementes des Bearbeitungselementes und der Siegelfläche des Gegenelementes ein Spalt bildet, in welchem ein zu bearbeitendes Material angeordnet werden kann, wobei in einer Schnittansicht senkrecht zur Längsachse des Bearbeitungselementes die Siegelfläche einen Schweißabschnitt aufweist, der zumindest abschnittsweise konkav gekrümmt ist.

Wie bereits oben angemerkt kann das Gegenelement eine Sonotrode und das Bearbeitungselement ein Amboss sein. Durch die abschnittsweise Krümmung des Schweißabschnittes verlängert sich die Kontaktzeit zwischen Sonotrode und Amboss, sodass mehr Energie in das Material eingebracht werden kann, wodurch ebenfalls die Vorschubgeschwindigkeit erhöht werden kann, ohne dass die Kraft mit welcher die Sonotrode auf das zu bearbeitende Material gedrückt wird, erhöht werden muss.

In einer weiteren bevorzugten Ausführungsform ist vorgesehen, dass der Krümmungsradius des konkav gekrümmten Abschnittes des Gegenelementes in etwa dem Krümmungsradius des Hauptabschnittes des Bearbeitungselementes entspricht. Tatsächlich ist es besonders bevorzugt, wenn der Krümmungsradius des konkav gekrümmten Abschnittes des Gegenelementes geringfügig größer als der Krümmungsradius des Hauptabschnittes des Bearbeitungselementes ist. Dabei entspricht die Differenz der beiden Krümmungsradien der Breite des Spaltes, der zwischen Siegelfläche und Oberseite des Strukturelementes während der schweißenden Bearbeitung verbleibt.

In einer weiteren Ausführungsform weist das Gegenelement Rillen zur zumindest teilweisen Aufnahme zumindest eines Fadens auf, die in einer Vorschubrichtung orientiert sind, in welcher das zu bearbeitende Material durch den Spalt zwischen Bearbeitungselement und Gegenelement hindurchbewegt wird, wobei das zu bearbeitende Material aus mindestens zwei Materialbahnabschnitten und dem zumindest einen Faden besteht, wobei der zumindest eine Faden zwischen den beiden Materialbahnabschnitten positioniert ist. Auf diese Weise können mit dem erfindungsgemäßen Bearbeitungselement in einer Ultraschallbearbeitungsvorrichtung auch raffbare Materialien hergestellt werden.

In einer weiteren bevorzugten Ausführungsform weist die Siegelfläche einen Einlaufabschnitt auf, der neben dem Schweißabschnitt angeordnet ist und entweder nicht gekrümmt ist oder mit einem Krümmungsradius konkav gekrümmt ist, welcher größer als der Krümmungsradius des Schweißabschnittes ist. Auch hier ändert sich am Übergang zwischen Einlaufabschnitt und Schweißabschnitt die Steigung oder die Krümmung der Siegelfläche, sodass im Bereich des Einlaufabschnittes der Abstand zwischen Bearbeitungselement und Gegenelement sukzessiv kleiner wird bis der kleinste Abstand erreicht wird, welcher dem Abstand zwischen Schweißabschnitt und Oberseite des Strukturelementes entspricht. Dieser Einlaufabschnitt ist derart angeordnet, dass ein in Vorschubrichtung durch den Spalt bewegtes Material zunächst mit dem Einlaufabschnitt und dann mit dem Schweißabschnitt in Kontakt kommt.

Dabei ist es von Vorteil, wenn der Einlaufabschnitt und der Schweißabschnitt in etwa gleich groß sind.

Weitere Vorteile, Merkmale und Anwendungsmöglichkeiten der vorliegenden Erfindung werden deutlich anhand der folgenden Beschreibung einer bevorzugten Ausführungsform und der zugehörigen Figuren.

Es zeigen:
- Figur 1: eine Ultraschallschweißvorrichtung in einer perspektivischen Ansicht,
- Figur 2: eine Detailvergrößerung des mit X gekennzeichneten Bereiches von Figur 1,
- Figur 3: eine Detailvergrößerung von Figur 2,
- Figur 4: eine Seitenansicht der Ultraschallschweißvorrichtung von Figur 1 und
- Figur 5: eine vergrößerte Teilansicht von Figur 4.
- Figur 6: eine schematische Darstellung einer weiteren Ausführungsform des erfindungsgemäßen Bearbeitungselementes.
- Figur 7: eine Vergrößerung der schematischen Darstellung in Figur 6.

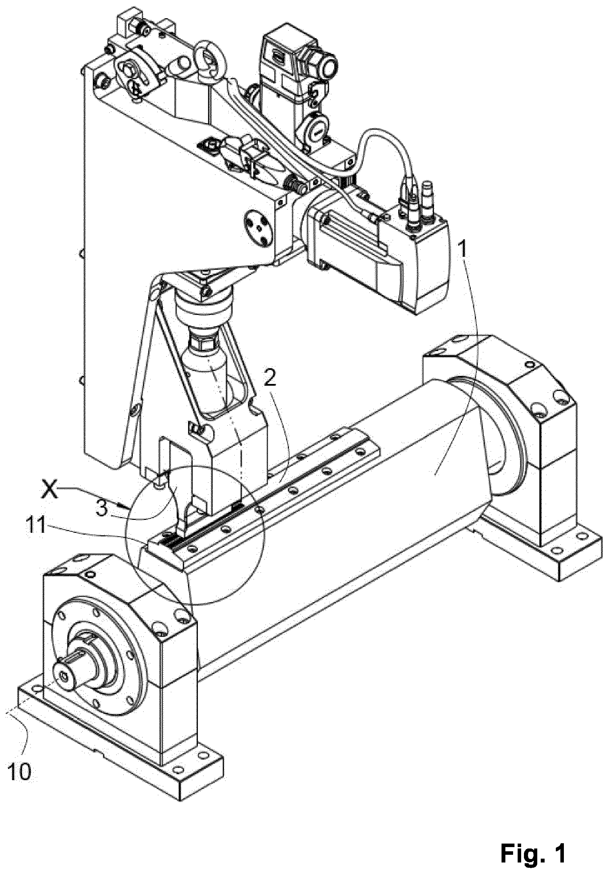

In Figur 1 ist eine perspektivische Ansicht einer Ultraschallschweißanlage gezeigt. Die Ultraschallschweißanlage weist ein als Amboss ausgebildetes Bearbeitungselement 1 auf, welches hier als Walze ausgeführt ist, die um eine Längsachse 10 drehbar ist. Auf der Walze ist mindestens eine Quernahtleiste 11 mit einer Trägerfläche 2 angeordnet. Gegenüberliegend ist ein als Sonotrode ausgebildetes Gegenelement 3 angeordnet.

Das Gegenelement 3 kann hier mit einer Ultraschallschwingung angeregt werden. Zu verarbeitendes Material wird dann zwischen der Trägerfläche 2 und der der Trägerfläche 2 zugewandten Siegelfläche der Sonotrode 3 hindurchbewegt, wobei die Bewegungsgeschwindigkeit des Materials mit der Umfangsgeschwindigkeit des Bearbeitungselementes 1 übereinstimmt. Dabei ist der Spalt zwischen Trägerfläche 2 und Sonotrode 3 so zu wählen, dass bei der Bearbeitung die Ultraschallschwingung auf das Material übertragen wird und es zu einem Aufschmelzen der thermoplastischen Bestandteile an den Grenzflächen kommt.

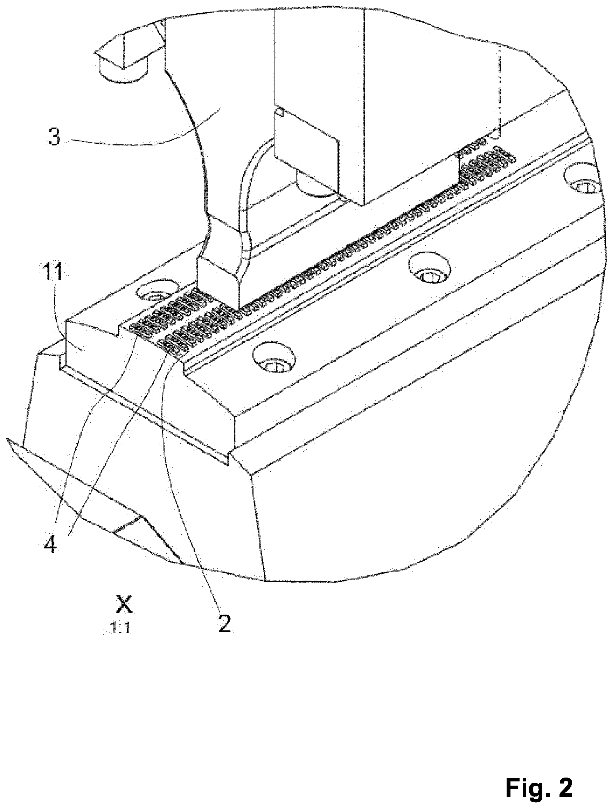

In Figur 2 ist eine Detailvergrößerung von Figur 1 gezeigt.

Man erkennt, dass auf der Trägerfläche 2 eine Vielzahl von Strukturelementen 4 angeordnet sind. Die Strukturelemente 4 haben eine längliche Form, die in Umfangsrichtung orientiert sind. Die Strukturelemente 4 treten bei der Bearbeitung mit dem Material in Kontakt und bestimmen das Schweißmuster, das bei der Bearbeitung in das Material eingebracht wird. Die Ultraschallschweißanlage kann beispielsweise zum Erstellen von Seitennähten von Windeln aus Vliesstoff verwendet werden.

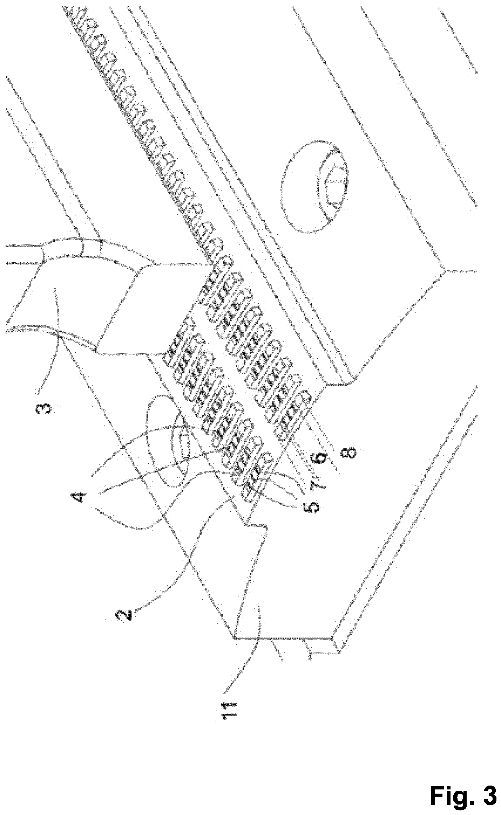

In Figur 3 ist eine Detailvergrößerung von Figur 2 dargestellt, in welcher die Strukturelemente 4 gut zu erkennen sind. In Umfangsrichtung (bezogen auf die Längsachse 10) sind zwei Strukturelemente 4 nebeneinander angeordnet. In axialer Richtung sind eine Vielzahl solcher Strukturelementpaare nebeneinander angeordnet.

Jedes Strukturelement weist einen Hauptabschnitt 6 auf sowie zwei Fasenabschnitte 7, 8, die gegenüber dem Hauptabschnitt 6 stärker gekrümmt sind. Im Hauptabschnitt 6 sind Rillen 5 eingebracht worden, die in der gezeigten Ausführungsform senkrecht zur Umfangsrichtung verlaufen. Es ist nicht notwendig, dass die Rillen senkrecht zur Umfangsrichtung verlaufen. Um den erfindungsgemäßen Effekt zu erzielen, sollten sie jedoch nicht parallel zur Umfangsrichtung angeordnet werden. Falls die Rillen doch parallel zur Umfangsrichtung angeordnet sind, sollten sie sich nicht über das gesamte Strukturelement 4 erstrecken.

Bei der schweißenden Bearbeitung rollen die Strukturelemente 4 auf dem zu bearbeitenden Material ab, sodass zunächst der Fasenabschnitt 8 mit dem zu bearbeitenden Material in Kontakt tritt. Aufgrund der abgewinkelten Anordnung des Fasenabschnittes 8 verringert sich in diesem Bereich der Abstand zwischen dem Strukturelement 4 und der gegenüber angeordneten Siegelfläche des Gegenelementes 3 sukzessive, bis der geringste Abstand im Bereich des Hauptabschnittes 6 erreicht wird. Der Hauptabschnitt 6 kann konvex gekrümmt ausgebildet sein, wobei der Krümmungsradius im Wesentlichen dem Abstand zwischen der Oberseite des Strukturelementes 4 und der Längsachse 10 des Bearbeitungselementes 1 entspricht.

In den Hauptabschnitt 6 sind die Rillen 5 mit einer Tiefe von 0,1 mm und einer Breite von 0,3 mm eingebracht worden. In die sich dadurch ergebenden Ausnehmungen kann geschmolzenes Material eindringen, sodass es im Wesentlichen an Ort und Stelle verbleibt und durch die Strukturelemente nicht aus der Fügezone gequetscht wird.

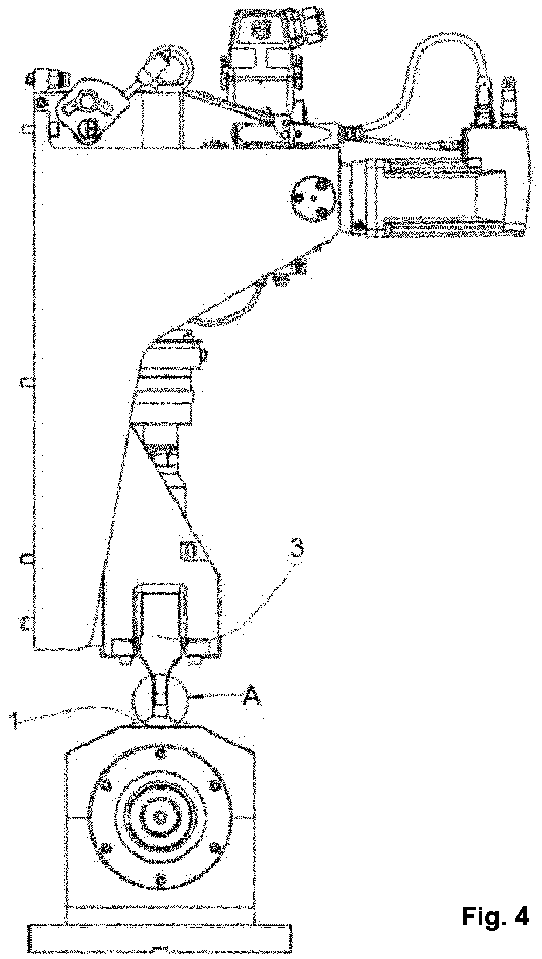

In Figur 4 ist eine Seitenansicht auf die Ultraschallschweißvorrichtung von Figur 1 gezeigt. Die Siegelfläche, d.h. die Fläche, die der Trägerfläche bzw. den Strukturelementen 4 zugewandt ist, ist die Fläche 9.

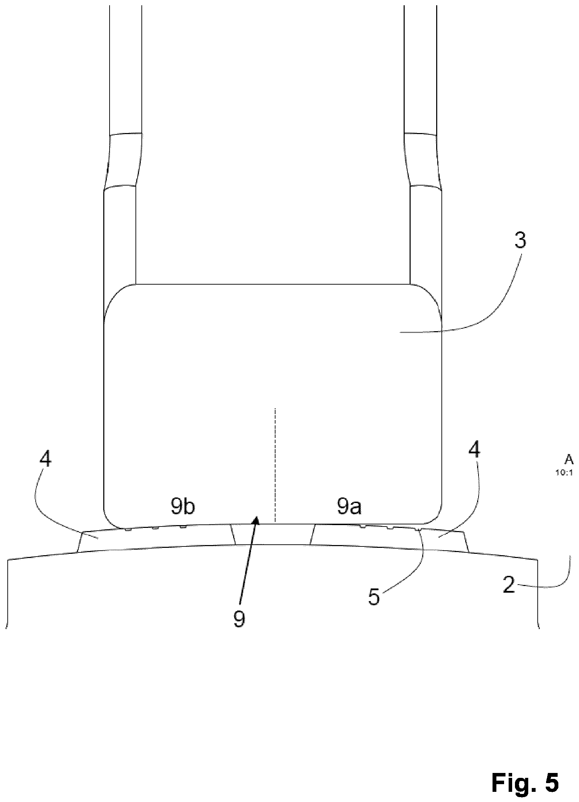

In Figur 5 ist eine vergrößerte Teilansicht der Figur 4 dargestellt. Die Fläche 9 besteht hier aus einem Einlaufabschnitt 9a und einem Schweißabschnitt 9b. Der Schweißabschnitt 9b ist konkav gekrümmt und zwar im Wesentlichen mit dem gleichen Krümmungsradius wie der Krümmungsradius des Hauptabschnittes des Bearbeitungselementes. Durch diese Maßnahme wird sichergestellt, dass während der Bearbeitung das Material länger mit der Sonotrode in Kontakt bleibt, sodass mehr Energie in das zu bearbeitende Material eingebracht werden kann. Der Einlaufabschnitt 9a ist in dieser Ausführungsform nicht gekrümmt ausgebildet und stellt dadurch sicher, dass das zu bearbeitende Material im Bereich des Einlaufabschnittes 9a zunächst in einen enger werdenden Spalt geführt wird. Im Bereich des Schweißabschnittes 9b ist der Spalt dann minimal und wird im Bereich des Schweißabschnittes im Wesentlichen konstant gehalten. Dabei erfolgt die Verschweißung hauptsächlich durch den Schweißabschnitt 9b, der Einlaufabschnitt 9a kann aber an seinem dem Schweißabschnitt 9b zugewandten Ende bereits zur Verschweißung beitragen.

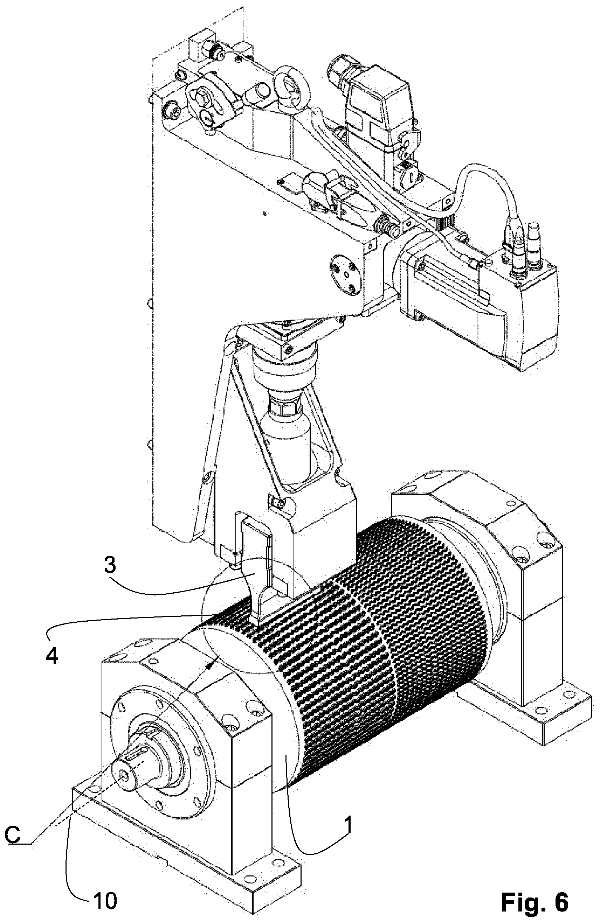

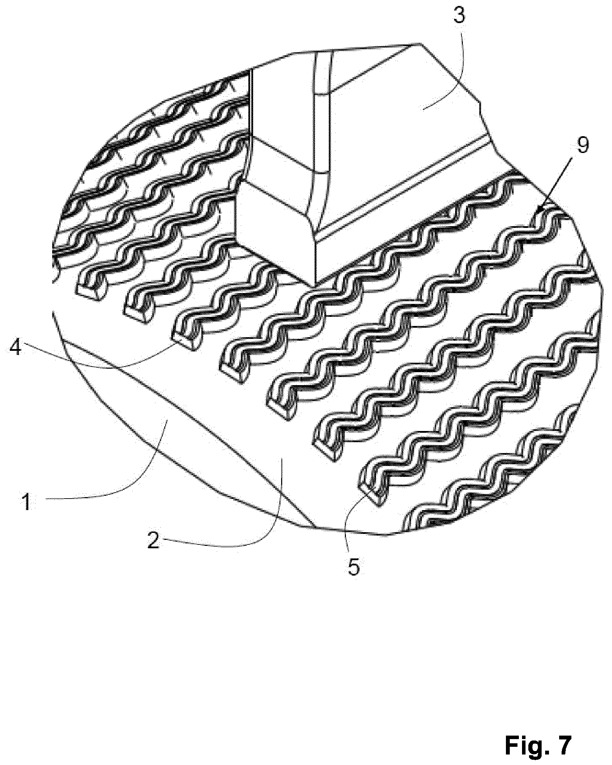

In den Figuren 6 und 7 ist schließlich eine alternative Ausführungsform des erfindungsgemä-βen Bearbeitungselementes 1 dargestellt, die sich insbesondere zur Herstellung raffbarer Materialien eignet. Dazu wird zwischen zwei Materialbahnabschnitten des zu bearbeitenden Materials mindestens ein Faden geführt, der durch die Strukturelemente 4 abschnittsweise kraft- oder stoffschlüssig mit den Materialbahnabschnitte verbunden wird. Die Strukturelemente 4 erstrecken sich dabei kontinuierlich über die gesamte Ausdehnung des Amboss 1 in Richtung der Längsachse 10. Weiter verlaufen das Strukturelement 4 und damit auch die Rillen 5 schlangenlinienförmig (siehe Figur 7). Durch die in den Figuren 6 und 7 gezeigte Ausführungsform wird der Faden stabil mit den Materialbahnschnitten verbunden, wobei gleichzeitig die Reibung zwischen Bearbeitungselement 1 und Material reduziert ist.

### Bezugszeichenliste

- 1: Bearbeitungselement (Amboss)
- 2: Trägerfläche
- 3: Gegenelement (Sonotrode)
- 4: Strukturelemente
- 5: Rillen
- 6: Hauptabschnitt
- 7, 8: Fasenabschnitte
- 9: Siegelfläche
- 9a: Einlaufabschnitt
- 9b: Schweißabschnitt
- 10: Längsachse
- 11: Quernahtleiste

## Patentansprüche

1. Bearbeitungselement (1) für die Bearbeitung eines Materials, wie z.B. eine Sonotrode oder ein Amboss, mit einer im Wesentlichen zylinderförmigen oder zylindersegmentförmigen Trägerfläche (2), die dafür vorgesehen ist, während der Bearbeitung mit dem Material in Kontakt zu treten, wobei das Bearbeitungselement (1) dafür vorgesehen ist, während der Bearbeitung um seine Längsachse gedreht zu werden, sodass die Trägerfläche (2) sich in einer Umfangsrichtung bewegt und auf dem zu bearbeitenden Material abrollt, wobei auf der Trägerfläche (2) zumindest ein Strukturelement (4) angeordnet ist, welches in radialer Richtung über der Trägerfläche (2) vorsteht, wobei das Strukturelement (4) eine Oberseite aufweist, die dafür vorgesehen ist, mit dem zu bearbeitenden Material in Kontakt zu treten, **dadurch gekennzeichnet, dass** die Oberseite einen Basisabschnitt und zumindest einen Ausnehmungsabschnitt (5) aufweist, welcher einen geringeren Abstand von der Längsachse (10) hat als der Basisabschnitt, wobei in einer Schnittansicht senkrecht zur Längsachse (10) Basisabschnitt und Ausnehmungsabschnitt (5) nebeneinander angeordnet sind, wobei die durch den Ausnehmungsabschnitt (5) gebildete Ausnehmung sich nicht bis zur Trägerfläche (2) erstreckt, wobei vorzugsweise die Ausnehmung eine Tiefe von weniger als 1 mm und besonders bevorzugt zwischen 0,05 mm und 0,2 mm hat.

2. Bearbeitungselement (1) nach Anspruch 1, **dadurch gekennzeichnet, dass** der Ausnehmungsabschnitt als Rille (5), welche vorzugsweise nicht in Umfangsrichtung ausgerichtet ist, ausgebildet ist.

3. Bearbeitungselement (1) nach Anspruch 2, **dadurch gekennzeichnet, dass** die Rille (5) eine Breite hat, die kleiner als 1 mm ist und vorzugsweise kleiner als 0,6mm ist und besonders bevorzugt zwischen 0,2 mm und 0,4 mm beträgt.

4. Bearbeitungselement (1) nach Anspruch 2 oder 3, **dadurch gekennzeichnet, dass** die Rille (5) eine Querschnittsfläche von weniger als 0,15 mm² hat, vorzugsweise von weniger als 0,05 mm² hat und besonders bevorzugt die Querschnittsfläche zwischen 0,015 mm² und 0,04 mm² beträgt.

5. Bearbeitungselement (1) nach einem Ansprüche 2 bis 4, **dadurch gekennzeichnet, dass** das Strukturelement (4) eine Mehrzahl von Rillen (5), vorzugsweise mindestens drei Rillen (5), in der Oberseite aufweist, welche nicht in Umfangsrichtung ausgerichtet sind, wobei vorzugsweise die Rillen (5) parallel zueinander angeordnet sind.

6. Bearbeitungselement (1) nach einem der vorherigen Ansprüche, **dadurch gekennzeichnet, dass** die Oberseite einen Hauptabschnitt (6), der im Wesentlichen eben oder mit einer konvexen Krümmung mit einem Krümmungsradius, der dem Abstand des Hauptabschnittes (6) von der Zylinderachse entspricht, ausgebildet ist, und mindestens einen sich an den Hauptabschnitt (6) in Umfangsrichtung anschließenden Fasenabschnitt (7,8) aufweist, der entweder gegenüber dem Hauptabschnitt (6) abgewinkelt ist, so dass Haupt- und Fasenabschnitt (6,7,8) einen Winkel < 180° einschließen, und/oder konvex gekrümmt ist, wobei, wenn der Hauptabschnitt (6) konvex gekrümmt ausgebildet ist, der Krümmungsradius des Fasenabschnittes (7,8) kleiner als der Krümmungsradius des Hauptabschnittes (6) ist,wobei vorzugsweise der mindestens eine Ausnehmungsabschnitt (5) im Hauptabschnitt (6) angeordnet ist.

7. Bearbeitungselement (1) nach einem der vorherigen Ansprüche, **dadurch gekennzeichnet, dass** mindestens zwei in Umfangsrichtung voneinander beabstandete Strukturelemente (4) vorgesehen sind.

8. Bearbeitungselement (1) nach einem der vorherigen Ansprüche, **dadurch gekennzeichnet, dass** es als Amboss (1) ausgebildet ist.

9. Bearbeitungselement (1) nach einem der vorherigen Ansprüche, **dadurch gekennzeichnet, dass** die Oberseite eine längliche Form mit einer Länge I und einer Breite b hat, wobei l>b ist.

10. Bearbeitungselement (1) nach eine der vorherigen Ansprüche, wobei sich das Strukturelement (4) sowie der in der Oberseite angeordnete Ausnahmeabschnitt (5) kontinuierlich über eine gesamte Länge l des Bearbeitungselements (1) erstreckt, wobei die Länge l im Wesentlichen parallel zu der Längsachse (10) orientiert ist.

11. Ultraschallschweißvorrichtung mit einem Bearbeitungselement (1) nach einem der vorherigen Ansprüche und einem Gegenelement (3), wobei das Gegenelement (3) eine Siegelfläche (9) aufweist, die gegenüberliegend zu dem Bearbeitungselement (1) angeordnet werden kann, so dass sich zwischen der Oberseite und der Siegelfläche (9) ein Spalt bildet, in welchem ein zu bearbeitendes Material angeordnet werden kann, wobei in einer Schnittansicht senkrecht zur Längsachse (10) des Bearbeitungselements (1) die Siegelfläche (9) einen Schweißabschnitt (9b) aufweist, der zumindest abschnittweise konkav gekrümmt ist, wobei vorzugsweise der Krümmungsradius des konkav gekrümmten Abschnittes des Gegenelementes (3) in etwa dem Krümmungsradius des Hauptabschnittes (6) des Bearbeitungselementes (1) entspricht.

12. Ultraschallschweißvorrichtung nach Anspruch 11, **dadurch gekennzeichnet, dass** das Gegenelement (3) Rillen zur zumindest teilweisen Aufnahme zumindest eines Fadens aufweist, die in einer Vorschubrichtung orientiert sind, in welcher das zu bearbeitende Material durch den Spalt zwischen Bearbeitungselement (1) und Gegenelement (3) hindurchbewegt wird, wobei das zu bearbeitende Material aus mindestens zwei Materialbahnabschnitten und dem zumindest einen Faden besteht, wobei der zumindest eine Faden zwischen den beiden Materialbahnabschnitten positioniert ist, wobei vorzugsweise das Bearbeitungselement (1) dafür vorgesehen ist, in einer Vorschubrichtung gedreht zu werden, in welcher ein zu bearbeitendes Material zwischen Bearbeitungselement (1) und Gegenelement (3) hindurchbewegt wird, wobei Einlaufabschnitt (9a) und Schweißabschnitt (9b) derart angeordnet sind, dass ein in Vorschubrichtung durch den Spalt bewegtes Material zunächst mit dem Einlaufabschnitt (9a) und dann mit dem Schweißabschnitt (9b) in Kontakt kommt.

13. Ultraschallschweißvorrichtung nach Anspruch 11, **dadurch gekennzeichnet, dass** die Siegelfläche (9) einen Einlaufabschnitt (9a) aufweist, der neben dem Schweißabschnitt (9b) angeordnet ist, und entweder nicht gekrümmt ist oder mit einem Krümmungsradius konkav gekrümmt ist, welcher größer als der Krümmungsradius des Schweißabschnittes (9b) ist.

14. Ultraschallschweißvorrichtung nach Anspruch 12 oder 13, **dadurch gekennzeichnet, dass** der Einlaufabschnitt (9a) und der Schweißabschnitt (9b) in etwa gleich groß sind.

15. Ultraschallschweißvorrichtung nach einem der Ansprüche 11 bis 14, **dadurch gekennzeichnet, dass** das Gegenelement (3) als Sonotrode ausgebildet ist.

## Claims

1. A processing element (1) for processing a material, such as a sonotrode or an anvil, comprising a substantially cylindrical or cylinder-segment-shaped carrier surface (2) which is intended to come into contact with the material during processing, the processing element (1) being intended to be rotated about its longitudinal axis during processing so that the carrier surface (2) moves in a circumferential direction and rolls on the material to be processed, at least one structural element (4) being arranged on the carrier surface (2), projecting in a radial direction above the carrier surface (2), the structural element (4) having an upper side intended to come into contact with the material to be processed, **characterized in that** the upper side has a base section and at least one recess section (5) which is at a smaller distance from the longitudinal axis (10) than the base section, wherein in a sectional view perpendicular to the longitudinal axis (10) the base section and recess section (5) are arranged next to one another, wherein the recess formed by the recess section (5) does not extend as far as the carrier surface (2), wherein preferably the recess has a depth of less than 1 mm and particularly preferably between 0.05 mm and 0.2 mm.

2. The processing element (1) according to claim 1, **characterized in that** the recess section is formed as a groove (5) which is preferably not aligned in the circumferential direction.

3. The processing element (1) according to claim 2, **characterized in that** the groove (5) has a width which is less than 1 mm and is preferably less than 0.6 mm and is particularly preferably between 0.2 mm and 0.4 mm.

4. The processing element (1) according to claim 2 or 3, **characterized in that** the groove (5) has a cross-sectional area of less than 0.15 mm², preferably less than 0.05 mm², and particularly preferably the cross-sectional area is between 0.015 mm² and 0.04 mm².

5. The processing element (1) according to any one of claims 2 to 4, **characterized in that** the structural element (4) has a plurality of grooves (5), preferably at least three grooves (5), in the top surface, which are not aligned in the circumferential direction, preferably the grooves (5) being arranged parallel to each other.

6. The processing element (1) according to any one of the preceding claims, **characterized in that** the top surface comprises a main section (6) which is substantially flat or with a convex curvature with a radius of curvature corresponding to the distance of the main section (6) from the cylinder axis, and at least one chamfer section (7, 8) adjoining the main section (6) in the circumferential direction, which chamfer section is either angled with respect to the main section (6), so that the main section and the chamfer section (6, 7, 8) form an angle < 180°, and/or is convexly curved, wherein, if the main section (6) is convexly curved, the radius of curvature of the chamfer section (7, 8) is smaller than the radius of curvature of the main section (6), wherein preferably the at least one recess section (5) is arranged in the main section (6).

7. The processing element (1) according to any one of the preceding claims, **characterized in that** at least two structural elements (4) spaced apart from one another in the circumferential direction are provided.

8. The processing element (1) according to any one of the preceding claims, **characterized in that** it is designed as an anvil (1).

9. The processing element (1) according to any one of the preceding claims, **characterized in that** the top surface has an elongate shape with a length I and a width b, where l>b.

10. The processing element (1) according to one of the preceding claims, wherein the structural element (4) as well as the recess section (5) arranged on the top surface extend continuously over an overall length I of the processing element (1), wherein the length I is substantially oriented parallel to the longitudinal axis (10).

11. An ultrasonic welding apparatus comprising a processing element (1) according to any one of the preceding claims and a counter-element (3), wherein the counter-element (3) having a sealing surface (9) which can be arranged opposite the processing element (1) so that a gap is formed between the top surface and the sealing surface (9) in which a material to be processed can be arranged, wherein, in a sectional view perpendicular to the longitudinal axis (10) of the processing element (1), the sealing surface (9) has a welding section (9b) which is concavely curved at least in sections, wherein preferably the radius of curvature of the concavely curved section of the counter-element (3) corresponds approximately to the radius of curvature of the main section (6) of the processing element (1).

12. The ultrasonic welding apparatus according to claim 11, **characterized in that** the counter-element (3) comprises grooves for at least partially receiving at least one thread, which grooves are oriented in a feed direction in which the material to be processed is moved through the gap between the processing element (1) and the counter-element (3), wherein the material to be processed consists of at least two material web sections and the at least one thread, wherein the at least one thread is positioned between the two material web sections, wherein preferably the processing element (1) is intended to be rotated in a feed direction in which a material to be processed is passed between the processing element (1) and the counter-element (3), wherein the inlet section (9a) and the welding section (9b) are arranged such that a material moved in the feed direction through the gap comes into contact first with the inlet section (9a) and then with the welding section (9b).

13. The ultrasonic welding apparatus according to claim 11, **characterized in that** the sealing surface (9) has an inlet section (9a) arranged adjacent to the welding section (9b) and is either non-curved or concavely curved with a radius of curvature which is larger than the radius of curvature of the welding section (9b).

14. The ultrasonic welding apparatus according to claim 12 or 13, **characterized in that** the inlet section (9a) and the welding section (9b) are approximately equal in size.

15. The ultrasonic welding apparatus according to any one of claims 11 to 14, **characterized in that** the counter-element (3) is designed as a sonotrode.

## Revendications

1. Elément d'usinage (1) pour usiner un matériau, tel que, par exemple, une sonotrode ou une enclume, avec une surface de support (2) ayant essentiellement la forme d'un cylindre ou d'un segment de cylindre, qui est configurée pour entrer en contact, pendant l'usinage, avec le matériau, l'élément d'usinage (1) étant configuré pour, pendant l'usinage, être mis en rotation autour de son axe longitudinal, de façon que la surface de support (2) soit en mouvement dans une direction circonférentielle et se déroule sur le matériau à usiner, au moins un élément de structure (4) étant disposé sur la surface de support (2) et dépassant de la surface de support (2) dans la direction radiale, l'élément de structure (4) comprenant une face supérieure qui est configurée pour entrer en contact avec le matériau à usiner, **caractérisé en ce que** la face supérieure comprend une partie de base et au moins une partie d'évidement (5) qui est moins espacée de l'axe longitudinal (10) que la partie de base, dans une vue en coupe perpendiculaire à l'axe longitudinal (10), la partie de base et la partie d'évidement (5) étant disposées l'une à côté de l'autre, l'évidement formé par la partie d'évidement (5) ne s'étendant pas jusqu'à la surface de support (2), l'évidement ayant de préférence une profondeur de moins de 1 mm et, de manière particulièrement préférée, une profondeur entre 0,05 mm et 0,2 mm.

2. Elément d'usinage (1) selon la revendication 1, **caractérisé en ce que** la partie d'évidement est réalisée comme une rainure (5) qui, de préférence, n'est pas orientée dans la direction circonférentielle.

3. Elément d'usinage (1) selon la revendication 2, **caractérisé en ce que** la rainure (5) a une largeur inférieure à 1 mm et, de préférence, inférieure à 0,6 mm et, de manière particulièrement préférée, entre 0,2 mm et 0,4 mm.

4. Elément d'usinage (1) selon la revendication 2 ou 3, **caractérisé en ce que** la rainure (5) a une section transversale de moins de 0,15 mm², de préférence inférieure à 0,05 mm² et que, de manière particulièrement préférée, la section transversale est entre 0,015 mm² et 0,04 mm².

5. Elément d'usinage (1) selon l'une des revendications 2 à 4, **caractérisé en ce que** l'élément de structure (4) comprend une pluralité de rainures (5), de préférence au moins trois rainures (5), sur la face supérieure, qui ne sont pas orientées dans la direction circonférentielle, les rainures (5) étant disposées, de préférence, parallèlement l'une à l'autre.

6. Elément d'usinage (1) selon l'une des revendications précédentes, **caractérisé en ce que** la face supérieure comprend une partie principale (6) qui est, de préférence, réalisée de façon plane ou a une courbure convexe avec un rayon de courbure qui correspond à la distance entre la partie principale (6) et l'axe de cylindre, ainsi qu'au moins une partie de chanfrein (7, 8) attenante, dans la direction circonférentielle, à la partie principale (6) et qui est soit angulaire par rapport à la partie principale (6), de façon que la partie principale et la partie de chanfrein (6, 7, 8) enferment un angle inférieur à 180°, soit convexe lorsque la partie principale (6) a une forme convexe, le rayon de courbure de la partie de chanfrein (7, 8) étant inférieure au rayon de courbure de la partie principale (6), ladite au moins une partie d'évidement (5) étant disposée, de préférence, dans la partie principale (6).

7. Elément d'usinage (1) selon l'une des revendications précédentes, **caractérisé en ce qu'**au moins deux éléments de structure (4), espacés l'un de l'autre dans la direction circonférentielle, sont prévus.

8. Elément d'usinage (1) selon l'une des revendications précédentes, **caractérisé en ce qu'**il est configuré comme enclume (1).

9. Elément d'usinage (1) selon l'une des revendications précédentes, **caractérisé en ce que** la face supérieure a une forme allongée avec une longueur 1 et une largeur b, où l>b.

10. Elément d'usinage (1) selon l'une des revendications précédentes, l'élément de structure (4) et la partie d'évidement (5) disposée sur la face supérieure s'étendant de manière continue sur une longueur totale 1 de l'élément d'usinage (1), la longueur 1 étant orientée essentiellement parallèlement à l'axe longitudinal (10).

11. Dispositif de soudage à ultrasons avec un élément d'usinage (1) selon l'une des revendications précédentes et avec un contre-élément (3), le contre-élément (3) comportant une surface de scellage (9) qui peut être disposée en face de l'élément d'usinage (1), si bien qu'une fente se forme entre la face supérieure et la surface de scellage (9) dans laquelle un matériau à usiner peut être disposé, la surface de scellage (9) comprenant, dans une vue en coupe perpendiculaire à l'axe longitudinal (10) de l'élément d'usinage (1), une partie de soudage (9b) qui est courbée, au moins par sections, de façon concave, le rayon de courbure de la partie courbée concave du contre-élément (3) correspondant de préférence en gros au rayon de courbure de la partie principale (6) de l'élément d'usinage (1).

12. Dispositif de soudage à ultrasons selon la revendication 11, **caractérisé en ce que** le contre-élément (3) comprend des rainures pour recevoir au moins partiellement au moins un fil, qui sont orientées dans une direction d'avancement dans laquelle le matériau à usiner est avancé à travers la fente entre l'élément d'usinage (1) et le contre-élément (3), le matériau à usiner étant constitué d'au moins deux parties de lé de matériau et dudit au moins un fil, ledit au moins un fil étant positionné entre les deux parties de lé de matériau, de préférence, l'élément d'usinage (1) étant conçu pour être tourné dans une direction d'avancement dans laquelle un matériau à usiner est déplacé entre l'élément d'usinage (1) et le contre-élément (3), la partie d'entrée (9a) et la partie de soudage (9b) étant disposées de façon qu'un matériau à usiner avancé à travers la fente dans la direction d'avancement, entre d'abord en contact avec la partie d'entrée (9a) et ensuite avec la partie de soudage (9b).

13. Dispositif de soudage à ultrasons selon la revendication 11, **caractérisé en ce que** la surface de scellage (9) comprend une partie d'entrée (9a) qui est disposée à côté de la partie de soudage (9b) et qui est soit non courbée ou est courbée de façon concave avec un rayon de courbure qui est supérieur au rayon de courbure de la partie de soudage (9b).

14. Dispositif de soudage à ultrasons selon la revendication 12 ou 13, **caractérisé en ce que** la partie d'entrée (9a) et la partie de soudage (9b) ont approximativement la même taille.

15. Dispositif de soudage à ultrasons selon l'une des revendications 11 à 14, **caractérisé en ce que** le contre-élément (3) est formé comme sonotrode.
